# EUROPEAN PATENT APPLICATION

(11) **EP 4 484 555 A1**
(43) Date of publication of application: **01.01.2025**
(21) Application number: 23756500.7
(22) Date of filing: 21.02.2023
(51) Int. Cl.: C12N 7/01, C12N 5/07

(54) **VIRAL VECTOR PRODUCIBILITY ENHANCER AND METHOD FOR PRODUCING VIRAL VECTOR**

(30) Priority: 21.02.2022 JP 2022025008
(71) Applicant: NIPPON MEDICAL SCHOOL FOUNDATION, Bunkyo-ku Tokyo 113-8602 (JP)
(72) Inventor: MIYAGAWA Yoshitaka, Tokyo 113-8602 (JP); OKADA Takashi, Tokyo 113-8602 (JP); KOBARI Yukage, Tokyo 113-8602 (JP); UCHIKAI Takao, Tokyo 104-0042 (JP)
(74) Representative: Mewburn Ellis LLP
(86) International application number: PCT/JP2023/006197
(87) International publication number: WO 2023/157976

(57) **Abstract**

This invention is intended to enhance production of viral vectors when producing viral vectors using viral vector-producing cells. A protein involved in the DNA damage response in viral vector-producing cells is inhibited.

## Description

### Technical Field

The present invention relates to an agent for enhancing production of viral vectors capable of enhancing production of viruses, which can be used in a process for producing viral vectors using cultured cells, and a method for producing viral vectors.

### Background Art

Gene therapy involving the use of viral vectors is a method of treatment comprising administering a viral vector comprising, integrated therein, a gene encoding a therapeutically effective protein to a patient or administering a cell comprising, introduced thereinto, such viral vector to a patient. Examples of viral vectors used in gene therapy include retrovirus, lentivirus, adenovirus, adeno-associated virus, Sendai virus, and herpesvirus vectors. While practical application of gene therapy has begun to date, there is an urgent need to develop a technique that enables efficient large-scale production of high-quality viral vectors in order to realize extensive application of gene therapy to a wide variety of diseases.

In general, viral vectors are produced by performing culture to proliferate vector-producing cells and then infecting the cells with a small amount of viruses. Thereafter, cell culture is continued to expand viral infection, and viral vectors are then proliferated in the virus-infected cells. The viral vectors proliferated in the cells are collected and purified in accordance with conventional techniques and then formulated, according to need.

In such a process of producing viral vectors, various attempts have been made in order to enhance production of viral vectors. For example, Patent Literature 1 discloses an agent that can improve efficiency for introducing a recombinant adeno-associated virus into a cell (i.e., transfection efficiency) in the process of producing viral vectors. The enhancing agent disclosed in Patent Literature 1 is valproic acid, a salt or a derivative thereof, isobutyric acid, a salt or a derivative thereof, or isovaleric acid, a salt or a derivative thereof.

Patent Literature 2 discloses an invention concerning a cell line used in a process of production of an adeno-associated viral vector in which expression of at least one of a gene encoding YB1 (also known as the Y box binding protein 1, the Y box transcription factor, or the nuclease sensitive element binding protein 1), a gene encoding NPM1 (also known as the nucleophosmin, the nucleolar phosphoprotein B23, or numatrin), and a gene encoding NCL (the nucleolar phosphoprotein nucleolin) is decreased. Use of the cell line disclosed in Patent Literature 2 leads to enhanced production of viral vectors.

In addition, Patent Literature 3 discloses an invention concerning a cell line used in a process of producing an adeno-associated viral vector into which a particular miRNA has been introduced. Use of the cell line disclosed in Patent Literature 3 enables production of adeno-associated viral vectors of higher titers than conventional viral vectors without complicated procedures.

### Citation List

### Patent Literature

Patent Literature 1: JP 2020-524498 A
Patent Literature 2: JP 2017-506885 A
Patent Literature 3: JP Patent No. 6,093,358

### Summary of Invention

### Technical Problem

The known techniques indicated above aimed at enhancing production of viral vectors; however, were not sufficient from the viewpoint of production of viral vectors. Accordingly, it remains necessary to develop a technique aimed at enhancing production of viral vectors.

Under the above circumstances, it is an object of the present invention to provide a novel agent for enhancing production of viral vectors that can enhance production of viral vectors and a method for producing viral vectors.

### Solution to Problem

The present inventors have conducted concentrated studies in order to attain the object described above. As a result, they discovered that production of viral vectors would be enhanced by inhibiting a protein involved in the DNA damage response in viral vector-producing cells. This has led to the completion of the present invention. The present invention encompasses the following.

(1) An agent for enhancing production of viral vectors comprising, as a major ingredient, an inhibitor against a protein involved in the DNA damage response.
(2) The agent for enhancing production of viral vectors according to (1), which is used for production of viral vectors using viral vector-producing cells.
(3) The agent for enhancing production of viral vectors according to (1), which comprises, as the inhibitor against a protein involved in the DNA damage response, one or more substances selected from the group consisting of an inhibitor against the sarcoplasmic reticulum Ca²⁺-ATPase, an inhibitor against the serine/threonine kinase Chk1, and an inhibitor against the Aurora kinase.
(4) The agent for enhancing production of viral vectors according to (3), wherein the inhibitor against the sarcoplasmic reticulum Ca²⁺ -ATPase is cyclopiazonic acid, Thapsigargin, or an analog thereof.
(5) The agent for enhancing production of viral vectors according to (3), wherein the inhibitor against the serine/threonine kinase Chk1 is PF-477736, Rabusertib, AZD7762, or an analog thereof or an RNAi molecule targeting the Chk1 gene.
(6) The agent for enhancing production of viral vectors according to (3), wherein the inhibitor against the Aurora kinase is CYC116, Alisertib, or an analog thereof or an RNAi molecule targeting the Aurora kinase gene.
(7) A method for producing viral vectors comprising: a step of culturing viral vector-producing cells in which a protein involved in the DNA damage response is inhibited; and a step of collecting viral vectors from the cultured cells.
(8) The method for producing viral vectors according to (7), wherein, in the step of culturing cells, the cells are cultured in a medium supplemented with the agent for enhancing production of viral vectors according to any of (1) to (6).
(9) The method for producing viral vectors according to (7), wherein the cells lack at least one gene selected from the group consisting of the sarcoplasmic reticulum Ca²⁺ -ATPase gene, the serine/threonine kinase Chk1 gene, and the Aurora kinase gene.

### Advantageous Effects of Invention

The present invention can provide an agent for enhancing production of viral vectors that can act on viral vector-producing cells to significantly enhance production of viral vectors from the cells. The present invention can also provide a method capable of significantly enhancing production of viral vectors, in a production of viral vectors using viral vector-producing cells.

### Brief Description of Drawings

[Figure 1] Figure 1 shows a chart demonstrating the results of measuring the effects of enhancing production of rAAV vectors achieved with the use of a chemical substance D (an inhibitor against the sarcoplasmic reticulum Ca²⁺-ATPase gene).
[Figure 2] Figure 2 shows a chart demonstrating the results of measuring the effects of enhancing production of rAAV vectors achieved with the use of a chemical substance D (an inhibitor against the sarcoplasmic reticulum Ca²⁺-ATPase gene) with the elapse of time.
[Figure 3] Figure 3 shows photographs demonstrating the results of the infectivity test of the rAAV vectors produced with the use of a chemical substance D (an inhibitor against the sarcoplasmic reticulum Ca²⁺-ATPase gene).
[Figure 4] Figure 4 shows a chart demonstrating the results of measuring the effects of enhancing production of rAAV vectors achieved with the use of a chemical substance D2 (an inhibitor against the sarcoplasmic reticulum Ca²⁺-ATPase gene).
[Figure 5] Figure 5 shows a chart demonstrating the results of measuring the effects of enhancing production of rAAV vectors achieved with the use of a chemical substance K (an inhibitor against the serine/threonine kinase Chk1).
[Figure 6] Figure 6 shows a chart demonstrating the results of measuring the effects of enhancing production of rAAV vectors achieved with the use of a chemical substance K (an inhibitor against the serine/threonine kinase Chk1) with the elapse of time.
[Figure 7] Figure 7 shows charts demonstrating the results of evaluating the AAV vector-producing ability by knocking down the human CHEK1 gene, which is a target molecule of a chemical substance K (an inhibitor against the serine/threonine kinase Chk1), by siRNA.
Figure 7 (A) shows the expression level of the human CHEK1 gene treated with siRNA targeting the human CHEK1 gene or no-target siRNA and Figure 7 (B) shows the amount of production of rAAV vectors when treated with siRNA targeting the human CHEK1 gene or no-target siRNA.
[Figure 8] Figure 8 shows a chart demonstrating the results of measuring the effects of enhancing production of rAAV vectors achieved with the use of a chemical substance K2 (an inhibitor against the serine/threonine kinase Chk1).
[Figure 9] Figure 9 shows a chart demonstrating the results of measuring the effects of enhancing production of rAAV vectors achieved with the use of a chemical substance K3 (an inhibitor against the serine/threonine kinase Chk1).
[Figure 10] Figure 10 shows a chart demonstrating the results of measuring the effects of enhancing production of rAAV vectors achieved with the use of a chemical substance L (an inhibitor against the Aurora kinase).
[Figure 11] Figure 11 shows charts demonstrating the results of evaluating the rAAV vector-producing ability by knocking down the human AURKA gene, which is a target molecule of a chemical substance L (an inhibitor against the Aurora kinase), by siRNA. Figure 11 (A) shows the expression level of the human AURKA gene treated with siRNA targeting human AURKA gene or no-target siRNA and Figure 11 (B) shows the amount of production of rAAV vectors when treated with siRNA targeting the human AURKA gene or no-target siRNA.
[Figure 12] Figure 12 shows a chart demonstrating the results of measuring the effects of enhancing production of rAAV vectors achieved with the use of a chemical substance L2 (an inhibitor against the Aurora kinase).
[Figure 13] Figure 13 shows a chart demonstrating the results of measuring the effects of enhancing production of rAAV vectors achieved with the use of siRNA targeting the human CHEK1 gene in combination with a chemical substance D (an inhibitor against the sarcoplasmic reticulum Ca²⁺-ATPase gene).
[Figure 14] Figure 14 shows a chart demonstrating the results of measuring the effects of enhancing production of rAAV vectors achieved with the use of siRNA targeting the human AURKA gene in combination with a chemical substance D (an inhibitor against the sarcoplasmic reticulum Ca²⁺-ATPase gene).
[Figure 15] Figure 15 shows a chart demonstrating the results of measuring the effects of enhancing production of rAAV vectors achieved with the use of a chemical substance L2 (an inhibitor against the Aurora kinase) in combination with a chemical substance K2 (an inhibitor against the serine/threonine kinase Chk1).
[Figure 16] Figure 16 shows a chart demonstrating the results of measuring the effects of enhancing production of rAAV vectors achieved with the use of a chemical substance D3 (an inhibitor against the sarcoplasmic reticulum Ca²⁺-ATPase gene).
[Figure 17] Figure 17 shows a chart demonstrating the results of measuring the effects of enhancing production of rAAV vectors achieved with the use of a chemical substance L3 (an inhibitor against the Aurora kinase).
[Figure 18] Figure 18 shows a chart demonstrating the results of measuring the effects of enhancing production of rAAV vectors achieved with the use of a chemical substance L4 (an inhibitor against the Aurora kinase).
[Figure 19] Figure 19 shows a chart demonstrating the results of measuring the effects of enhancing production of rAAV vectors achieved with the use of a chemical substance L5 (an inhibitor against the Aurora kinase).
[Figure 20] Figure 20 shows charts demonstrating the results of evaluating the rAAV vector-producing ability by knocking down the human AURKB gene, which is a target molecule of a chemical substance L (an inhibitor against the Aurora kinase), by siRNA. Figure 20 (A) shows the expression level of the human AURKB gene treated with siRNA targeting the human AURKB gene or no-target siRNA and Figure 20 (B) shows the amount of production of rAAV vectors when treated with siRNA targeting the human AURKB gene or no-target siRNA.
[Figure 21] Figure 21 shows a chart demonstrating the results of measuring the effects of enhancing production of rAAV vectors achieved with the use of ascorbic acid.

### Description of Embodiments

The agent for enhancing production of viral vectors and the method for producing viral vectors according to the present invention enhance production of viral vectors using viral vector-producing cells.

In an embodiment of the present invention, a protein involved in the DNA damage response in viral vector-producing cells is inhibited, so as to enhance production of viral vectors. The term "production of viral vectors" used herein indicates that the amount of viral vectors is larger than that of the control when the cells are cultured under given conditions or the term indicates that the virus titer is higher than that of the control when the cells are cultured under given conditions. The amount of viral vectors or the virus titer can be adequately measured in accordance with conventional techniques. Examples of techniques for measuring the amount of viral vectors or the virus titer include, but are not particularly limited to, plaque assay, TCID50 assay (the tissue culture infectious dose), and quantitative RT-PCR assay.

In the present invention, viral vectors are viruses produced and amplified by artificial means and genetically modified viruses for research and medical purposes. Types of viruses are not particularly limited, and examples of viruses include: non-enveloped viruses, such as adeno-associated virus (AAV), adenovirus, enterovirus, parvovirus, papovavirus, human papillomavirus, rotavirus, Coxsackie virus, Sapovirus, norovirus, poliovirus, echovirus, hepatitis A virus, hepatitis E virus, rhinovirus, astrovirus, circovirus, and simian virus; and enveloped viruses, such as retrovirus, lentivirus, Sendai virus, herpesvirus such as herpes simplex virus, vaccinia virus, measles virus, baculovirus, influenza virus, leukemia virus, Sindbis virus, and poxvirus. Among them, a viral vector used for gene therapy, such as the adeno-associated virus, is preferably used.

An adeno-associated virus is a member of the Parvoviridae virus family that packages a linear single-stranded DNA in a capsid. Examples of adeno-associated viruses include AAV type 1 (AAV1), AAV type 2 (AAV2), AAV type 3 (AAV3), AAV type 4 (AAV4), AAV type 5 (AAV5), AAV type 6 (AAV6), AAV type 7 (AAV7), AAV type 8 (AAV8), AAV type 9 (AAV9), and AAV type 10 (AAV10). Examples also include artificially prepared AAV viral vectors, such as AAV-DJ and AAV-PHP.B. Such viral vectors can be applied to techniques for packaging and amplifying artificial nucleic acids in adeno-associated viral capsids.

The term "viral vector-producing cell" is synonymous with a packaging cell that is capable of producing the viral vectors. An example thereof is a cell resulting from transfection of a viral vector into the human embryonic kidney 293 (HEK293) cell (i.e., a packaging cell). A cell to be transfected with a viral vector is not limited to the HEK293 cell, and examples thereof include HEK293T cell, HEK293S cell, HEK293F cell, HEK293FT cell, HEK293FTM cell, HEK293 SG cell, HEK293 SGGD cell, HEK293H cell, HEK293E cell, and HEK293MSR cell derived from the HEK293 cell. A commercially available cell developed for the adenovirus (e.g., the Adeno-X 293 cell line), a commercially available cell developed for the adeno-associated virus (e.g., the AAVpro 293T cell line), and the like can also be used. Examples of other mammal-derived packaging cells include human cervical cancer-derived HeLa cell, human lung cancer-derived A549 cell, human fibrosarcoma HT-1080 cell, human retinal pigment epithelium-derived cell, such as the PER. C6 cell, hamster-derived BHK and CHO cells, and African green monkey-derived Vero and COS cells.

A technique of producing viral vectors is not limited to a method for transfecting plasmids into packaging cells, and examples of techniques include a method of infecting packaging cells with helper viruses, such as adenoviruses or herpesviruses, to amplify the viruses and a method of integrating a constitutive factor for packaging viral vectors into the cell genome to establish and amplify producer cells.

Viral vector-producing cells can be used for techniques that amplify viral vectors using insect-derived cells in addition to mammal-derived cells. Examples of such techniques include a method of amplification comprising transfection of viral vectors with the use of lepidopteran insect-derived cells represented by Sf9 and Sf21 cells derived from the ovary of the cabbage armyworm and Tni cells and High Five cells derived from *Trichoplusia ni*, and a method of amplification comprising infection of the cells with the baculovirus comprising a constitutive factor for packaging viral vectors mounted thereon.

A medium in which the viral vector-producing cells are cultured is not particularly limited, and an adequate medium can be selected in accordance with the cells to be used. Examples of media that can be used include the Dulbecco's Modified Eagle Medium supplemented with 10% fetal bovine serum (DMEM supplemented with 10% FBS or DMEM supplemented with 10% FCS) and the serum-free Eagle Minimum Essential Medium (E-MEM). Viral vector-producing cells may be cultured by adhesion culture or suspension culture.

### [Inhibition of protein involved in DNA damage response]

In the present invention, a protein involved in the DNA damage response is selected from the group consisting of the sarcoplasmic reticulum Ca²⁺ -ATPase, the serine/threonine kinase Chk1, and the Aurora kinase. As a result of inhibition of at least one of such proteins, production of viral vectors in the cells indicated above can be enhanced. "Inhibition of a protein" encompasses both of an embodiment in which an inhibitor against the protein is allowed to react with the viral vector-producing cells and an embodiment in which cells in which functions of the protein are suppressed are used as the viral vector-producing cells. The cells in which functions of the protein are suppressed can be obtained by, for example, deleting an endogenous gene encoding the protein.

Examples of inhibitors against the proteins include substances that inhibits production and/or activity of the protein and substances that promote degradation and/or inactivation of the protein. Substances that inhibit production of the protein are not particularly limited. For example, production of the protein may be inhibited by destroying DNA encoding the protein and a regulatory sequence thereof or introducing a mutation with the use of a substance such as an RNAi molecule targeting DNA encoding the protein, a ribozyme, an antisense nucleic acid, a DNA/RNA chimeric polynucleotide, a vector expressing any thereof, or a dominant negative mutant against the protein, and a method such as a genome editing technique represented by gene targeting or CRISPR.

As a substance that inhibits the protein of interest, any compound that reacts with the protein can be used. Examples of compounds that can be used include organic compounds (e.g., an amino acid, a polypeptide or a derivative thereof, a low-molecular-weight compound, a sugar, and a polymer compound) and inorganic compounds. Such compound may be a natural substance or an unnatural substance. Examples of polypeptide derivatives include a modified polypeptide obtained with the addition of a modifying group and a variant polypeptide obtained by modifying an amino acid residue. Such compound may be a single compound, or it may be, for example, a compound library, an expression product of a gene library, a cell extract, a cell culture supernatant, a fermented microbial product, a marine creature extract, or a plant extract.

Among proteins involved in the DNA damage response, more specifically, the sarcoplasmic reticulum Ca²⁺ -ATPase is encoded by the SERCA, it is present in the endoplasmic reticulum membrane of muscle cells, and it is involved in a calcium ion pump that transports calcium ions to the endoplasmic reticulum. Examples of inhibitors against the sarcoplasmic reticulum Ca²⁺ -ATPase include cyclopiazonic acid, 2,5-di-tert-butyl-1,4-benzohydroquinone, gingerol, CGP 37157, ruthenium red, t-butylhydroquinone, Thapsigargin, paxillin, linoleic acid amide, Suramin, saikosaponin D, and an analog of any of such compounds. As the inhibitor against the sarcoplasmic reticulum Ca²⁺-ATPase, in particular, use of cyclopiazonic acid having the structure indicated below, which is expressed as a chemical substance D in the present specification, or an analog thereof, is preferable.

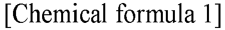

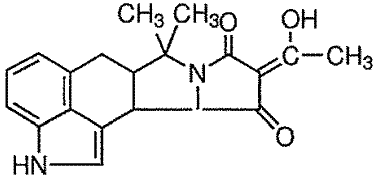

An analog of cyclopiazonic acid is a compound having a structure derived from the structure indicated above by substitution of a given atom or atoms with another atom or other atoms, which has inhibitory activity against the sarcoplasmic reticulum Ca²⁺ -ATPase as with cyclopiazonic acid.

When an inhibitor against the sarcoplasmic reticulum Ca²⁺ -ATPase, such as cyclopiazonic acid, is used as an agent for enhancing production of viral vectors, the concentration thereof in a medium in which viral vector-producing cells are cultured is not particularly limited. For example, the concentration can be 5 to 60 µM. The concentration is preferably 10 to 50 µM, more preferably 20 to 50 µM, and further preferably 20 to 40 µM.

As the inhibitor against the sarcoplasmic reticulum Ca²⁺-ATPase, use of Thapsigargin having the structure indicated below, which is expressed as a chemical substance D2 in the present specification, or an analog thereof, is preferable.

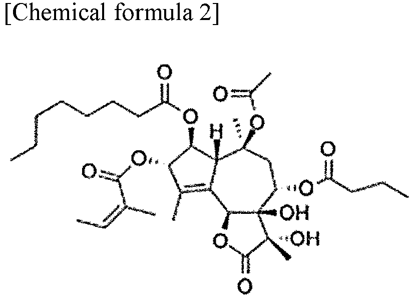

An analog of Thapsigargin is a compound having a structure derived from the structure indicated above by substitution of a given atom or atoms with another atom or other atoms, which has inhibitory activity against the sarcoplasmic reticulum Ca²⁺ -ATPase as with Thapsigargin.

When an inhibitor against the sarcoplasmic reticulum Ca²⁺ -ATPase, such as Thapsigargin, is used as an agent for enhancing production of viral vectors, the concentration thereof in a medium in which viral vector-producing cells are cultured is not particularly limited. For example, the concentration can be 50 to 1000 nM. The concentration is preferably 100 to 1000 nM, more preferably 150 to 500 nM, and further preferably 200 to 400 nM.

As the inhibitor against the sarcoplasmic reticulum Ca²⁺ -ATPase, use of Suramin (suramin sodium salt) having the structure indicated below, which is expressed as a chemical substance D3 in the present specification, or an analog thereof, is preferable.

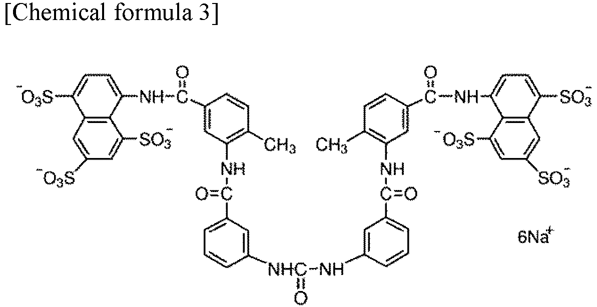

An analog of Suramin is a compound having a structure derived from the structure indicated above by substitution of a given atom or atoms with another atom or other atoms, which has inhibitory activity against the sarcoplasmic reticulum Ca²⁺ -ATPase as with Suramin.

When an inhibitor against the sarcoplasmic reticulum Ca²⁺ -ATPase, such as Suramin, is used as an agent for enhancing production of viral vectors, the concentration thereof in a medium in which viral vector-producing cells are cultured is not particularly limited. For example, the concentration can be 100 to 1000 µg/ml. The concentration is preferably 200 to 800 µg/ml, more preferably 400 to 800 µg/ml, and further preferably 500 to 700 µg/ml.

Among proteins involved in the DNA damage response, the serine/threonine kinase Chk1 is encoded by the CHEK1 gene, and it has activity of regulating the DNA damage response and the cell cycle checkpoint response. Examples of inhibitors against the serine/threonine kinase Chk1 include PF-477736, AZD7762, Rabusertib (LY2603618), MK-8776 (SCH 900776), CHIR-124, Prexasertib (LY2606368), VX-803 (M4344), DB07268, GDC-0575 (ARRY-575), Chk2 inhibitor II (BML-277), CCT245737, SAR-020106 and PD0166285, debromohymenialdisine, SB218078, TCS2312, PV1019, and analogs of such compounds. As the inhibitor against the serine/threonine kinase Chk1, in particular, use of PF-477736 having the structure indicated below, which is expressed as a chemical substance K in the present specification, or an analog thereof, is preferable.

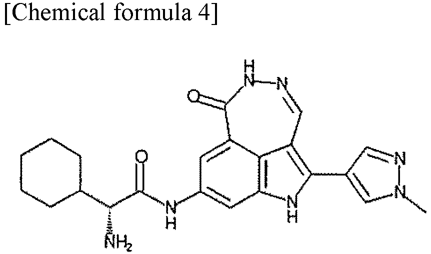

An analog of PF-477736 is a compound having a structure derived from the structure indicated above by substitution of a given atom or atoms with another atom or other atoms, which has inhibitory activity against the serine/threonine kinase Chk1 as with PF-477736.

When PF-477736 is used as an agent for enhancing production of viral vectors, the concentration thereof in a medium in which viral vector-producing cells are cultured is not particularly limited. For example, the concentration can be 100 nM to 4 µM. The concentration is preferably 150 nM to 3 µM, more preferably 300 nM to 3 µM, and further preferably 300 nM to 1.5 µM.

As the inhibitor against the serine/threonine kinase Chk1, use of Rabusertib having the structure indicated below, which is expressed as a chemical substance K2 in the present specification, or an analog thereof, is preferable.

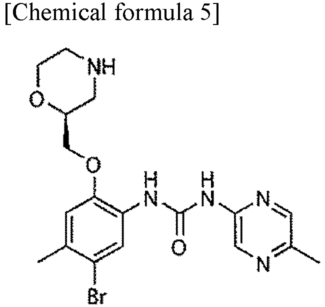

An analog of Rabusertib is a compound having a structure derived from the structure indicated above by substitution of a given atom or atoms with another atom or other atoms, which has inhibitory activity against the serine/threonine kinase Chk1 as with Rabusertib.

When Rabusertib is used as an agent for enhancing production of viral vectors, the concentration thereof in a medium in which viral vector-producing cells are cultured is not particularly limited. For example, the concentration can be 0.5 to 25 µM. The concentration is preferably 1 to 20 µM, and more preferably 1 to 5 µM.

As the inhibitor against the serine/threonine kinase Chk1, use of AZD7762 having the structure indicated below, which is expressed as a chemical substance K3 in the present specification, or an analog thereof, is preferable.

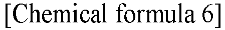

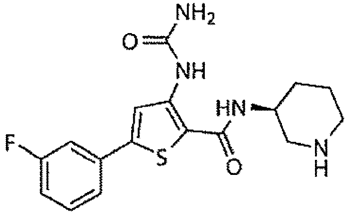

An analog of AZD7762 is a compound having a structure derived from the structure indicated above by substitution of a given atom or atoms with another atom or other atoms, which has inhibitory activity against the serine/threonine kinase Chk1 as with Rabusertib.

When AZD7762 is used as an agent for enhancing production of viral vectors, the concentration thereof in a medium in which viral vector-producing cells are cultured is not particularly limited. For example, the concentration can be 20 to 400 nM. The concentration is preferably 50 to 200 nM, and more preferably 50 to 150 nM.

Among proteins involved in the DNA damage response, the Aurora kinase comprises the Aurora kinase A encoded by the AURKA gene and the Aurora kinase B encoded by the AURKB gene, and the Aurora kinase has serine/threonine kinase activity. The inhibitor against the Aurora kinase may inhibit both or either of the Aurora kinase A and the Aurora kinase B. Alternatively, both of an inhibitor against the Aurora kinase that selectively inhibits the Aurora kinase A and an inhibitor against the Aurora kinase that selectively inhibits the Aurora kinase B may be used.

Examples of inhibitors against the Aurora kinase include CYC116, Alisertib (MI,N8237), Tozasertib (VX-680, MK-0457), SP600125, Barasertib (AZD1152-HQPA), ZM447439, MLN8054, Danusertib (PHA-739358), AT9283, JNJ-7706621, Hesperadin, Aurora A inhibitor I (TC-S 7010), KW-2449, SNS-314, ENMD-2076, PHA-680632, MK-5108 (VX-689), AMG-900, CCT129202, GSK1070916, TAK-901, CCT137690, MK-8745, ENMD-2076 L- (+)-tartaric acid, H-1152 dihydrochloride, XL228, LY3295668, Aurora kinase inhibitor III, SNS-314 mesylate, BI-847325, Reversine, SP-96, and analogs of these compounds. As the inhibitor against the Aurora kinase, in particular, use of CYC116 having the structure indicated below, which is expressed as a chemical substance L in the present specification, or an analog thereof, is preferable.

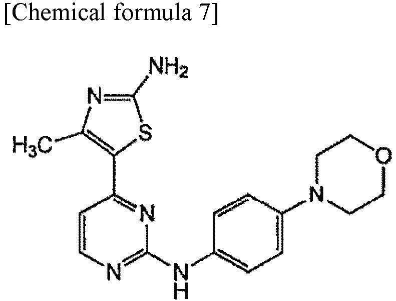

An analog of CYC116 is a compound having a structure derived from the structure indicated above by substitution of a given atom or atoms with another atom or other atoms, which has inhibitory activity against the Aurora kinase as with CYC116. It should be noted that inhibitory activity of CYC116 against the Aurora kinase is inhibitory activity against AURKA and AURKB.

When CYC116 is used as an agent for enhancing production of viral vectors, the concentration thereof in a medium in which viral vector-producing cells are cultured is not particularly limited. For example, the concentration can be 0.3 to 5 µM. The concentration is preferably 0.5 to 2 µM, more preferably 0.75 to 2 µM, and further preferably 0.75 to 1.25 µM.

As the inhibitor against the Aurora kinase, use of Alisertib having the structure indicated below, which is expressed as a chemical substance L2 in the present specification, or an analog thereof, is preferable.

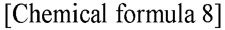

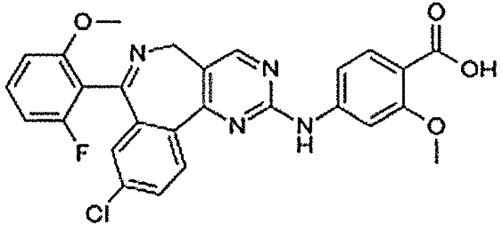

An analog of Alisertib is a compound having a structure derived from the structure indicated above by substitution of a given atom or atoms with another atom or other atoms, which has inhibitory activity against the Aurora kinase as with Alisertib. It should be noted that inhibitory activity of Alisertib against the Aurora kinase is specific inhibitory activity against AURKA.

When Alisertib is used as an agent for enhancing production of viral vectors, the concentration thereof in a medium in which viral vector-producing cells are cultured is not particularly limited. For example, the concentration can be 100 to 1600 nM. The concentration is preferably 200 to 800 nM, more preferably 250 to 600 nM, and further preferably 300 to 500 nM.

As the inhibitor against the Aurora kinase, use of Barasertib having the structure indicated below, which is expressed as a chemical substance L3 in the present specification, or an analog thereof, is preferable.

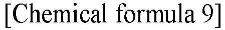

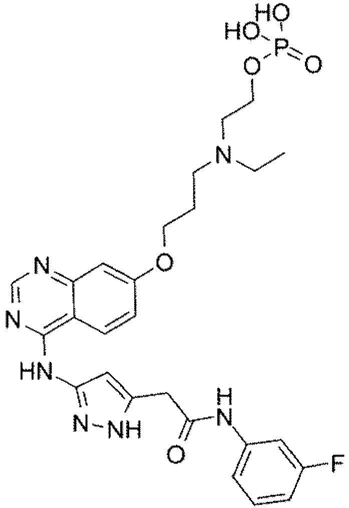

An analog of Barasertib is a compound having a structure derived from the structure indicated above by substitution of a given atom or atoms with another atom or other atoms, which has inhibitory activity against the Aurora kinase as with Barasertib. It should be noted that inhibitory activity of Barasertib against the Aurora kinase is specific inhibitory activity against AURKB.

When Barasertib is used as an agent for enhancing production of viral vectors, the concentration thereof in a medium in which viral vector-producing cells are cultured is not particularly limited. For example, the concentration can be 0.5 to 50 µM. The concentration is preferably 1 to 25 µM, more preferably 1 to 10 µM, and further preferably 2 to 4 µM.

As the inhibitor against the Aurora kinase, use of SP-96 having the structure indicated below, which is expressed as a chemical substance L4 in the present specification, or an analog thereof, is preferable.

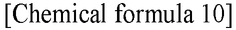

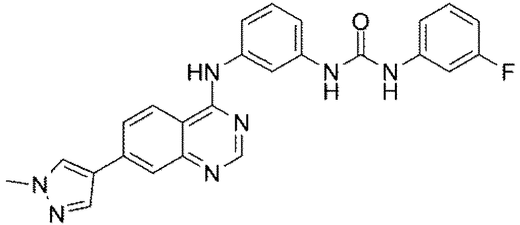

An analog of SP-96 is a compound having a structure derived from the structure indicated above by substitution of a given atom or atoms with another atom or other atoms, which has inhibitory activity against the Aurora kinase as with SP-96. It should be noted that inhibitory activity of SP-96 against the Aurora kinase is specific inhibitory activity against AURKB.

When SP-96 is used as an agent for enhancing production of viral vectors, the concentration thereof in a medium in which viral vector-producing cells are cultured is not particularly limited. For example, the concentration can be 100 to 2000 nM. The concentration is preferably 250 to 1000 nM, more preferably 250 to 750 nM, and further preferably 300 to 600 nM.

As the inhibitor against the Aurora kinase, use of MLN8054 having the structure indicated below, which is expressed as a chemical substance L5 in the present specification, or an analog thereof, is preferable.

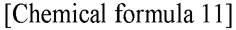

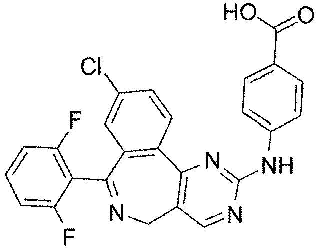

An analog of MLN8054 is a compound having a structure derived from the structure indicated above by substitution of a given atom or atoms with another atom or other atoms, which has inhibitory activity against the Aurora kinase as with MLN8054. It should be noted that inhibitory activity of MLN8054 against the Aurora kinase is inhibitory activity against AURKA and AURKB.

When MLN8054 is used as an agent for enhancing production of viral vectors, the concentration thereof in a medium in which viral vector-producing cells are cultured is not particularly limited. For example, the concentration can be 0.2 to 10 µM. The concentration is preferably 0.5 to 8 µM, and more preferably 1 to 4 µM.

### Examples

Hereafter, the present invention is described in greater detail with reference to the examples, although the technical scope of the present invention is not limited to the examples below.

### [Example 1]

### [Method of experiment]

### 1) Cell culture

The cells of the immortalized cell line derived from the human embryonic kidney (HEK293 cells) purchased from ATCC (American Type Culture Collection) were cultured in E-MEM supplemented with 10% fetal bovine serum, penicillin, and streptomycin at 37°C in the presence of 5% CO₂.

### 2) Preparation of AAV type 1 vector (rAAV1)

The recombinant adeno-associated virus 1 (rAAV1) was prepared with the use of 3 types of plasmids: pAAV-ZsGreen1 (TaKaRa Bio), pAAV2/1, and pHelper. A mixture of 3 types of plasmids for preparation of rAAV1 was adjusted to bring the mixing ratio of DNA to Polyethylenimine Max (PEI: Polysciences) (DNA:PEI) to 1:2 in OPTI-MEM, and the reaction was allowed to proceed at room temperature for 15 minutes. After 90% or higher confluent HEK293 cells were transfected with E-MEM supplemented with 10% FBS for 6 hours, culture was performed in serum-free E-MEM to prepare the AAV type 1 vector.

### 3) Measurement of virus titer by quantitative PCR (qPCR)

Genome DNA of rAAV1 was extracted to measure the virus titer. Specifically, the culture supernatant was collected and treated with benzonase at 37°C for 1 hour, and the viral genome DNA was extracted and purified using the DNeasy Blood & Tissue kit (QIAGEN). The virus titer was measured by real-time PCR in triplicate. qPCR was performed using primers targeting Zs-Green 1.

### 4) Evaluation of AAV vector-producing ability by treatment with chemical substance

The HEK293 cells were seeded at 4 × 10⁵ cells/well on a 24-well plate, plasmids for preparation of AAV vectors were introduced on the following day, the transfection solution was removed 6 hours later, the medium was exchanged with serum-free E-MEM supplemented with chemical substances at various concentrations as shown in Table 1, and culture was then performed for 3 days. In the present example, chemical substances D and D2, which are the inhibitors against the sarcoplasmic reticulum Ca²⁺ -ATPase, chemical substances K, K2, and, K3, which are the inhibitors against the serine/threonine kinase Chk1, and chemical substances L and L2, which are the inhibitors against the Aurora kinase, were tested. The virus titers achieved with the use of the chemical substances were compared with the virus titer of the control cells treated with serum-free E-MEM not supplemented with chemical substances. Changes in the amount of AAV production of the chemical substances D and K were observed with the elapse of time for 4 to 7 days.

**[Table 1]**

| | Chemical substance used | Manufacturer | Concentration |
|---|---|---|---|
| Chemical substance D | Cyclopiazonic acid | R&D Systems | 10, 20, 30, 40, 50 µM |
| Chemical substance D2 | Thapsigargin | EMD Millipore | 50, 100, 200, 300, 400, 500, 1000 nM |
| Chemical substance K | PF-477736 | Cayman Chemical | 150, 300, 450, 600, 1500, 3000 nM |
| Chemical substance K2 | Rabusertib (LY2603618) | Selleckchem.com | 1, 2, 3, 5, 10 µM |
| Chemical substance K3 | AZD7762 | Selleckchem.com | 20, 50, 100, 200, 400 nM |
| Chemical substance L | CYC116 | ChemScene LLC | 0.5, 0.75, 1, 1.25, 1.5, 2 µM |
| Chemical substance L2 | Alisertib (MLN8237) | Selleckchem.com | 100, 200, 400, 800, 1600 nM |

### 5) Evaluation of AAV vector quality by treatment with chemical substance

The quality of the AAV vectors produced from the HEK293 cells treated with chemical substances was evaluated in terms of purity and the infectious ability by SDS-PAGE.

### 6) Evaluation of infectious ability

The HEK293 cells were seeded at 4 × 10⁵ cells/well on a 24-well plate, plasmids for preparation of AAV vectors were transfected thereinto, and the resultant was then treated with chemical substances for 5 days. The culture supernatant was then collected. The HEK293 cells that were not treated with chemical substances were employed as the control cells. The virus titers were measured by qPCR, the HEK293 cells seeded at 6.25 × 10³ cells/well on a 96-well plate were transduced at 5 × 10⁴ v.g./cell, and, 3 days thereafter, a green fluorescence was observed and the image thereof was obtained under an inverted research microscope.

### 7) Evaluation of AAV vector-producing ability by siRNA

The target molecule of the tested chemical substance was knocked down by siRNA to evaluate the AAV vector-producing ability. At the outset, siRNA targeting the target molecule was purchased (Dharmacon siGENOME Human CHEK1 siRNA-SMARTpool, siGENOME Human AURKA siRNA-SMARTpool, Horizon Discovery Group Company) and then transfected into the HEK293 cells seeded at 3 × 10⁵ cells/well on a 12-well plate using a transfection solution (DharmaFECT 1 Transfection Reagent, Horizon Discovery Group Company). The final concentration of siRNA was adjusted to 25 nM. The cells were collected 48 hours after transfection, total RNA was extracted, cDNA was synthesized using the RNeasy Plus Mini Kit (QIAGEN), and real-time PCR was performed using the Applied Biosystems High-Capacity cDNA Reverse Transcription Kit (ThermoFisher) and the TB Green Premix Ex TaqII (TaKaRa Bio) through a cycle of 95°C for 5 seconds and 60°C for 34 seconds repeated 40 times to assay the target molecule.

The target molecule of the chemical substance K is the human CHEK1 gene, and the target molecule of the chemical substance L is the human AURKA gene. Thus, PCR was performed using primer sequences targeting human CHEK1 (forward primer: 5'-TGACTTCCGGCTTTCTAAGG-3' (SEQ ID NO: 1); reverse primer: 5'-TGTGGCAGGAAGCCAAAC-3' (SEQ ID NO: 2)) and primer sequences targeting human AURKA (forward primer: 5'-GGAGCCTTGGAGTTCTTTGC-3' (SEQ ID NO: 3); reverse primer: 5'-CCTCTGGCTGGGATTATGCT-3' (SEQ ID NO: 4)). As the internal standards for correction, primer sequences targeting human 18s rRNA (forward primer: 5'-GCGGCGGAAAATAGCCTTTG-3' (SEQ ID NO: 5); reverse primer: 5'-GATCACACGTTCCACCTCATC-3' (SEQ ID NO: 6)) were used. As the control cells, the HEK293 cells transfected with non-target siRNA (siGENOME Non-Targeting siRNA #5, Horizon Discovery Group Company) were used. The cells were compared to determine the knock down efficiency. The calibration curve used in PCR was prepared by extracting total RNA from the non-treated HEK293 cells, synthesizing cDNA therefrom, and diluting the resultant to 2-fold. Based on the determined knock down efficiency, the optimal conditions for the concentration of siRNA to be used, the number of cells to be seeded, and the amount of the transfection solution were determined. siRNA was introduced into the HEK293 cells seeded on a 24-well plate, AAV vector-producing plasmids were introduced thereinto 24 hours later, culture was performed for 3 days, the supernatant was collected, and the virus titer was then measured.

### [Results of experiment]

### 1) Chemical substances D (cyclopiazonic acid) and D2 (Thapsigargin)

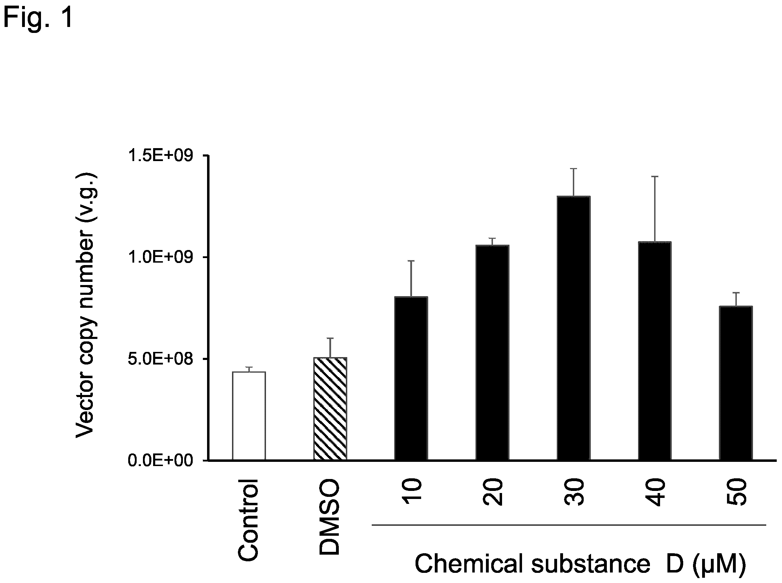

Figure 1 shows the results of measuring the effects of enhancing production of rAAV vectors achieved with the use of a chemical substance D. In Figure 1, the vertical axis represents the amount of production of AAV vectors (v.g.), and the horizontal axis represents the concentration of the chemical substance. As shown in Figure 1, the chemical substance D has effects of enhancing the amount of production of AAV vectors, and, in particular, greater effects of enhancing production of AAV vectors are observed in a range of concentration from 20 to 40 µM.

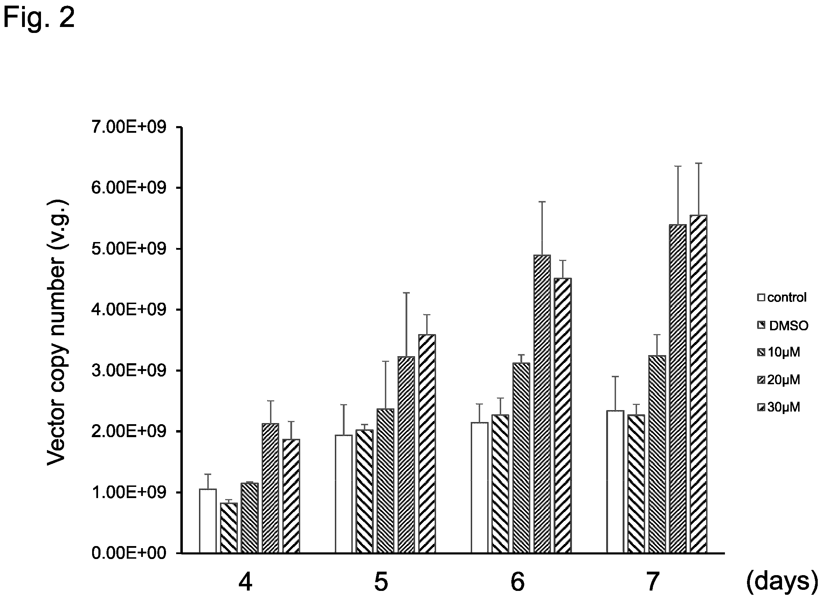

Figure 2 shows the results of measuring the effects of enhancing production of rAAV vectors achieved with the use of a chemical substance D with the elapse of time. In Figure 2, the vertical axis represents the amount of production of AAV vectors (v.g.), and the horizontal axis represents the time elapsed after the initiation of vector production (days). As shown in Figure 2, the amount of production of AAV vectors is increasing with the elapse of time after the initiation of vector production, and, in particular, the amount of production of AAV vectors is increasing 7 days after the treatment.

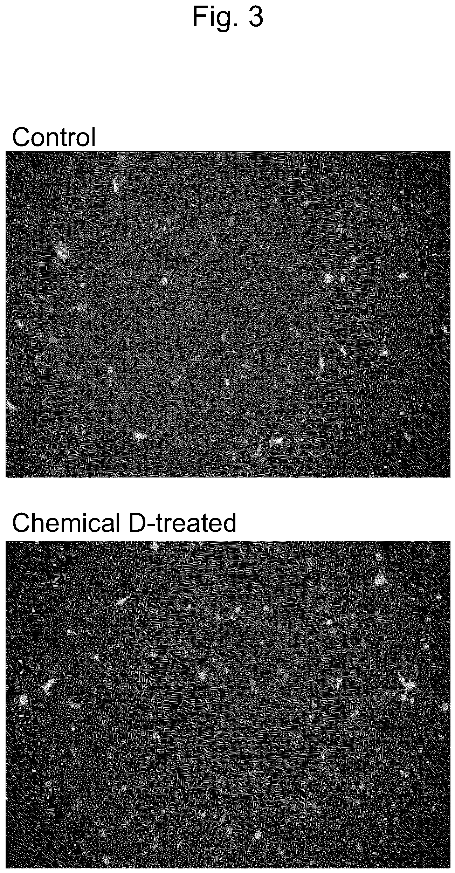

Figure 3 shows the results of the infectivity test of the rAAV vectors produced with the use of a chemical substance D. As shown in Figure 3, the rAAV vector produced by treatment with the chemical substance D retains the infectious ability equivalent to that of the AAV vector prepared without treatment with the chemical substance D.

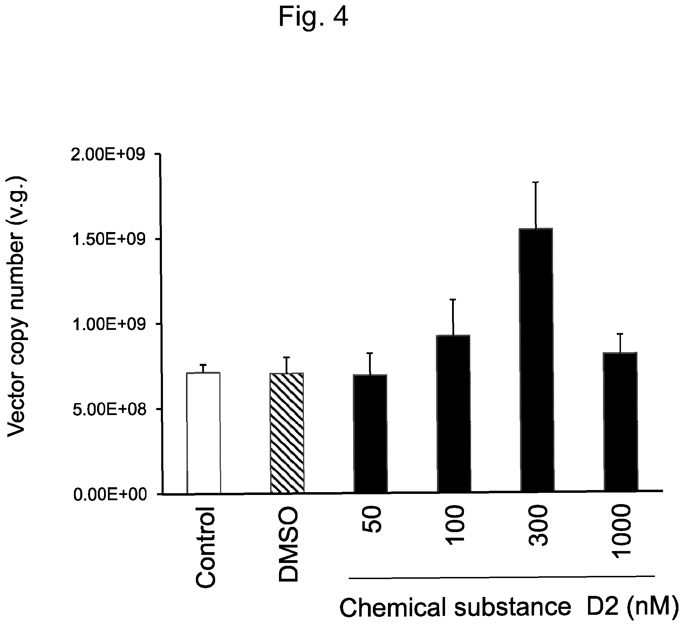

Figure 4 shows the results of measuring the effects of enhancing production of rAAV vectors achieved with the use of a chemical substance D2 (Thapsigargin) that inhibits the sarcoplasmic reticulum Ca²⁺ -ATPase as with the chemical substance D. In Figure 4, the vertical axis represents the amount of production of AAV vectors (v.g.), and the horizontal axis represents the concentration of the chemical substance. As shown in Figure 4, the chemical substance D2 has effects of enhancing the amount of production of AAV vectors, and, in particular, greater effects of enhancing production of AAV vectors are observed in a range of concentration from 100 to 1000 nM.

The results demonstrate that effects of enhancing production of rAAV vectors can be achieved through a reaction with the inhibitor against the sarcoplasmic reticulum Ca²⁺-ATPase, such as a chemical substance D or D2.

### 2) Chemical substances K (PF-477736), K2 (Rabusertib), and K3 (AZD7762)

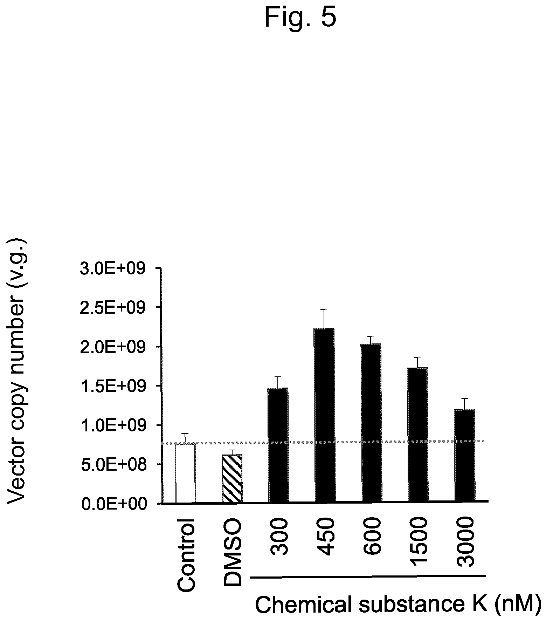

Figure 5 shows the results of measuring the effects of enhancing production of rAAV vectors achieved with the use of a chemical substance K. In Figure 5, the vertical axis represents the amount of production of AAV vectors (v.g.), and the horizontal axis represents the concentration of the chemical substance. As shown in Figure 5, the chemical substance K has effects of enhancing the amount of production of AAV vectors, and, in particular, greater effects of enhancing production of AAV vectors are observed in a range of concentration from 300 to 3000 nM.

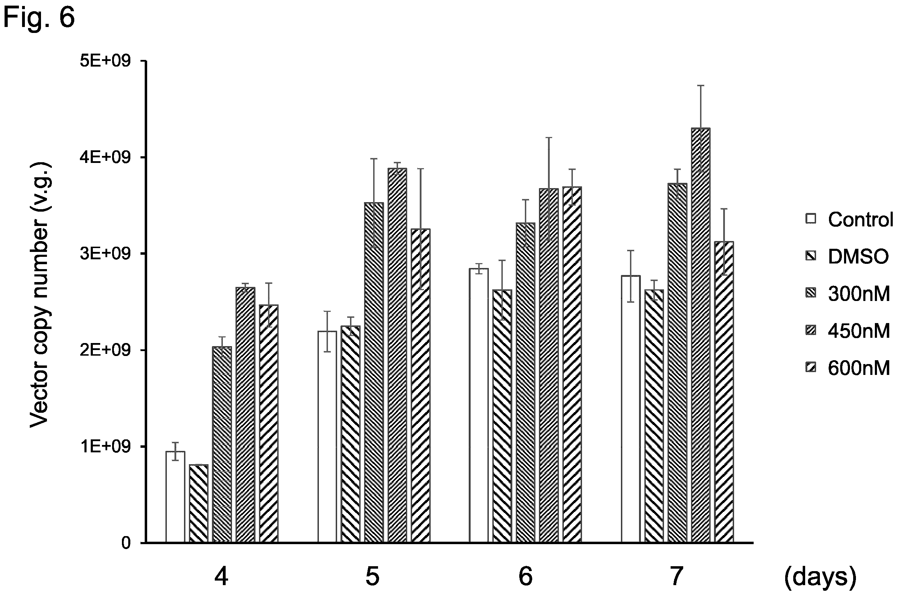

Figure 6 shows the results of measuring the effects of enhancing production of rAAV vectors achieved with the use of a chemical substance K with the elapse of time. In Figure 6, the vertical axis represents the amount of production of AAV vectors (v.g.), and the horizontal axis represents the time elapsed after the initiation of vector production (days). As shown in Figure 6, the amount of production of AAV vectors is increasing with the elapse of time after the initiation of vector production, and, in particular, the amount of production of AAV vectors is increasing 7 days after the treatment.

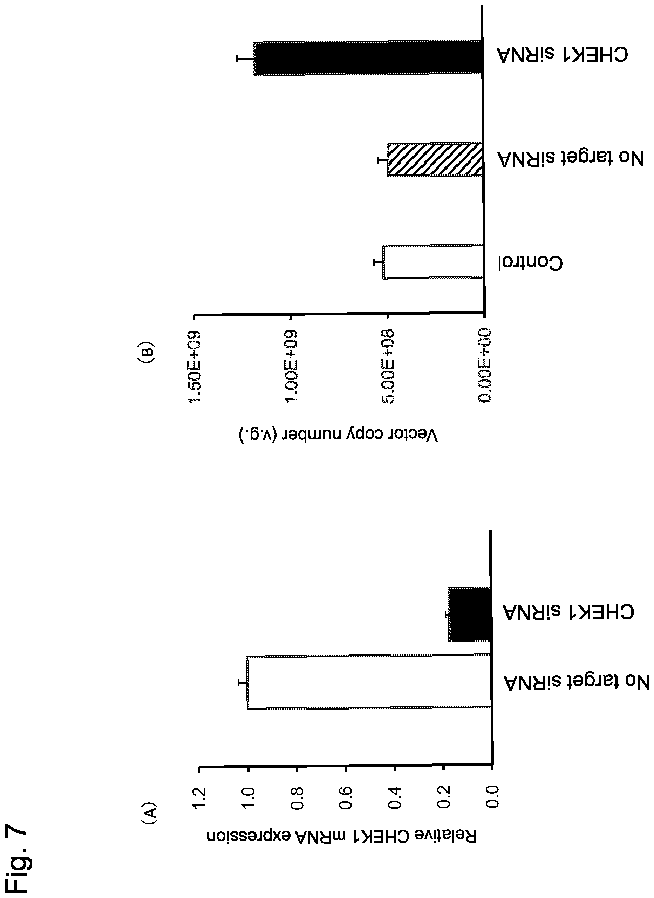

Figure 7 shows the results of evaluating the AAV vector-producing ability by knocking down the human CHEK1 gene, which is a target molecule of a chemical substance K, by siRNA. Figure 7 (A) shows the expression level of the human CHEK1 gene treated with siRNA targeting the human CHEK1 gene or no-target siRNA and Figure 7 (B) shows the amount of production of rAAV vectors when treated with siRNA targeting the human CHEK1 gene or no-target siRNA. As shown in Figure 7, effects of enhancing the amount of production of AAV vectors were achieved by knocking down the human CHEK1 gene, which is the target molecule of the chemical substance K, by siRNA. The results shown in Figure 7 demonstrate that the effects of enhancing production of rAAV vectors achieved with the use of the chemical substance K are achieved by inhibiting the target molecule of the chemical substance K and that effects of enhancing production of rAAV vectors can be achieved by inhibiting the human CHEK1 gene other than the chemical substance K.

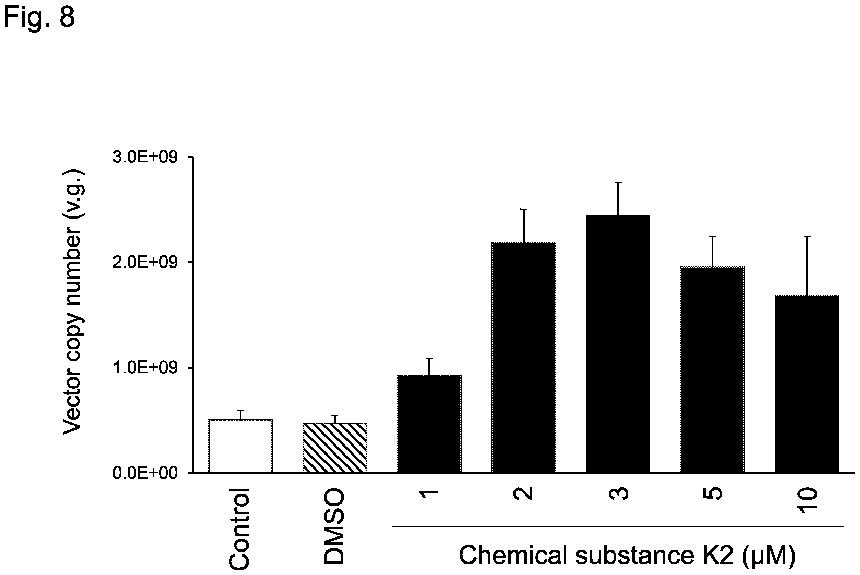

Figure 8 shows the results of measuring effects of enhancing production of rAAV vectors achieved with the use of a chemical substance K2 (Rabusertib) that inhibits human CHEK1 as with the chemical substance K. In Figure 8, the vertical axis represents the amount of production of AAV vectors (v.g.), and the horizontal axis represents the concentration of the chemical substance. As shown in Figure 8, the chemical substance K2 has effects of enhancing the amount of production of AAV vectors as with the chemical substance K, and, in particular, greater effects of enhancing production of AAV vectors are observed at the concentration of 3 µM.

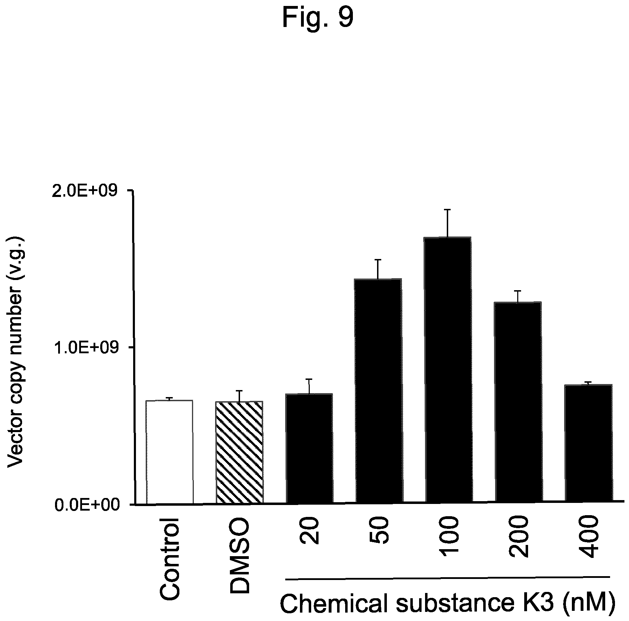

Figure 9 shows the results of measuring effects of enhancing production of rAAV vectors achieved with the use of a chemical substance K3 (AZD7762) that inhibits human CHEK1. In Figure 9, the vertical axis represents the amount of production of AAV vectors (v.g.), and the horizontal axis represents the concentration of the chemical substance. As shown in Figure 9, the chemical substance K3 has effects of enhancing the amount of production of AAV vectors as with the chemical substance K, and, in particular, greater effects of enhancing production of AAV vectors are observed in a range of concentration from 50 to 200 nM.

The results demonstrate that effects of enhancing production of rAAV vectors can be achieved through the reaction with the inhibitor against the serine/threonine kinase Chk1, such as a chemical substance K, K2, or K3.

### 3) Chemical substances L (CYC116) and L2 (Alisertib)

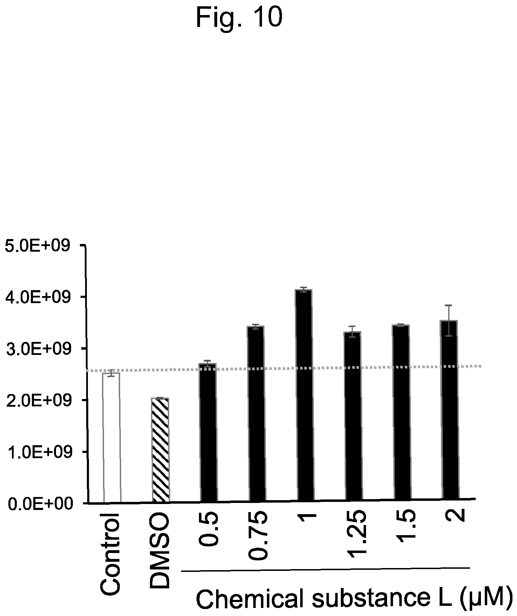

Figure 10 shows the results of measuring the effects of enhancing production of rAAV vectors achieved with the use of a chemical substance L. In Figure 10, the vertical axis represents the amount of production of AAV vectors (v.g.), and the horizontal axis represents the concentration of the chemical substance. As shown in Figure 10, the chemical substance L having inhibitory activity against AURKA and AURKB has effects of enhancing the amount of production of AAV vectors, and, in particular, greater effects of enhancing production of AAV vectors are observed in a range of concentration from 0.75 to 2 µM.

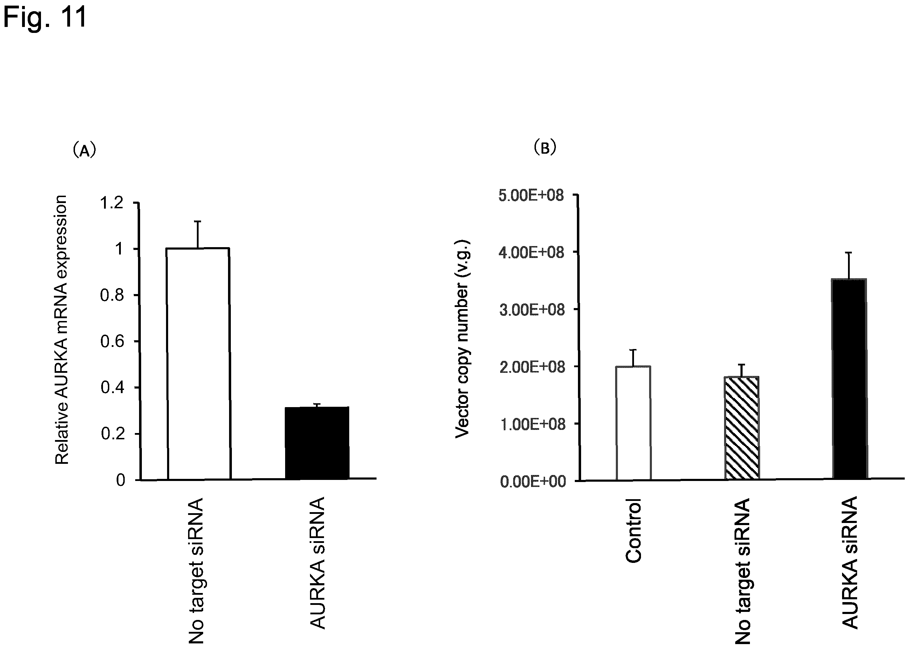

Figure 11 shows the results of evaluating the AAV vector-producing ability by knocking down the human AURKA gene, which is a target molecule of a chemical substance L, by siRNA. Figure 11 (A) shows the expression level of the human AURKA gene treated with siRNA targeting the human AURKA gene or no-target siRNA and Figure 11 (B) shows the amount of production of rAAV vectors when treated with siRNA targeting the human AURKA gene or no-target siRNA. As shown in Figure 11, effects of enhancing production of AAV vectors were achieved by knocking down the human AURKA gene, which is the target molecule of the chemical substance L, by siRNA. The results shown in Figure 11 demonstrate that the effects of enhancing the amount of production of rAAV vectors achieved with the use of the chemical substance L are achieved by inhibiting the target molecule of the chemical substance L and that effects of enhancing production of rAAV vectors can be achieved by inhibiting the human AURKA gene other than the chemical substance L.

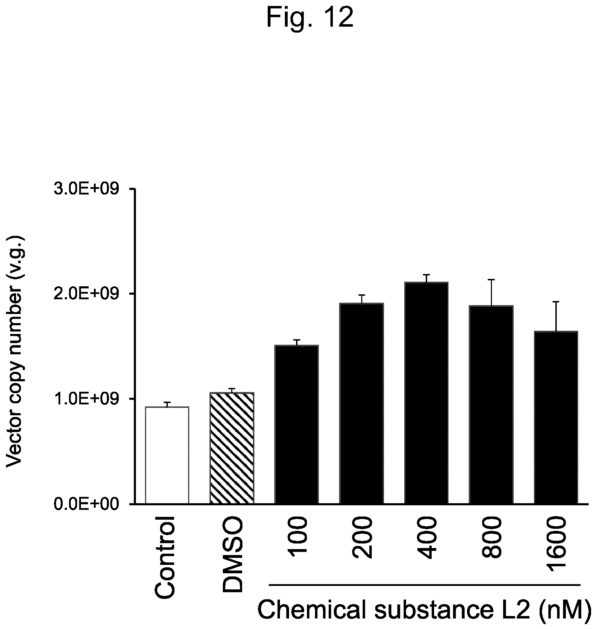

Figure 12 shows the results of measuring effects of enhancing production of rAAV vectors achieved with the use of a chemical substance L2 (Alisertib) that inhibits the Aurora kinase as with the chemical substance L. In Figure 12, the vertical axis represents the amount of production of AAV vectors (v.g.), and the horizontal axis represents the concentration of the chemical substance. As shown in Figure 12, the chemical substance L2 having specific inhibitory activity against AURKA has effects of enhancing the amount of production of AAV vectors as with the chemical substance L, and, in particular, greater effects of enhancing production of AAV vectors are observed in a range of concentration from 100 to 1600 nM.

### [Example 2]

In the present example, effects of enhancing production of AAV vectors achieved with the use of a plurality of types of chemical substances found to have effects of enhancing production of AAV vectors in Example 1 in combination were examined.

### [Combination Example 1]

The present example examines a combination of an inhibitor against the serine/threonine kinase Chk1 and an inhibitor against the sarcoplasmic reticulum Ca²⁺ -ATPase. As the inhibitor against the serine/threonine kinase Chk1, siRNA targeting the human CHEK1 gene was used. As the inhibitor against the sarcoplasmic reticulum Ca²⁺ -ATPase, a chemical substance D (cyclopiazonic acid) was used.

### [Combination Example 2]

The present example examines a combination of an inhibitor against the Aurora kinase and an inhibitor against the sarcoplasmic reticulum Ca²⁺ -ATPase. As the inhibitor against the Aurora kinase, siRNA targeting the human AURKA gene was used. As the inhibitor against the sarcoplasmic reticulum Ca²⁺ -ATPase, a chemical substance D (cyclopiazonic acid) was used.

### [Combination Example 3]

The present example examines a combination of an inhibitor against the Aurora kinase and an inhibitor against the serine/threonine kinase Chk1. As the inhibitor against the Aurora kinase, a chemical substance L2 (Alisertib) was used. As the inhibitor against the serine/threonine kinase Chk1, a chemical substance K2 (Rabusertib) was used.

### [Method of experiment]

At the outset, siGENOME Human CHEK1 siRNA (Dharmacon) or siGENOME Human AURKA siRNA or siGENOME Non-Targeting siRNA (Dharmacon) was introduced into the HEK293 cells seed on a 24-well plate, and, 24 hours thereafter, plasmids for preparation of AAV vectors were introduced in the same manner as in Example 1. The medium was exchanged with serum-free E-MEM supplemented with the chemical substance D at 20 µM or serum-free E-MEM not supplemented with the chemical substance 6 hours later, culture was performed for 3 days, and the virus titers were compared in the same manner as in Example.

In Combination Example 3, the HEK293 cells were seeded on a 24-well plate, and plasmids for preparation of AAV vectors were introduced on the following day. The transfection solution was removed 6 hours later, the medium was exchanged with serum-free E-MEM supplemented with the chemical substance K2 at 3 µM and the chemical substance L2 at 400 nM, culture was performed for 3 days, and the virus titers were compared between the group subjected to treatment with a single type of a chemical substance and the group not subjected to treatment.

### [Results of experiment]

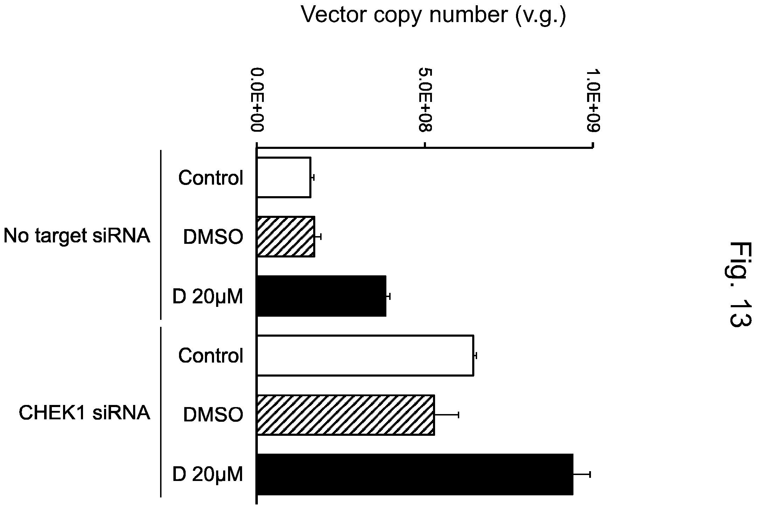

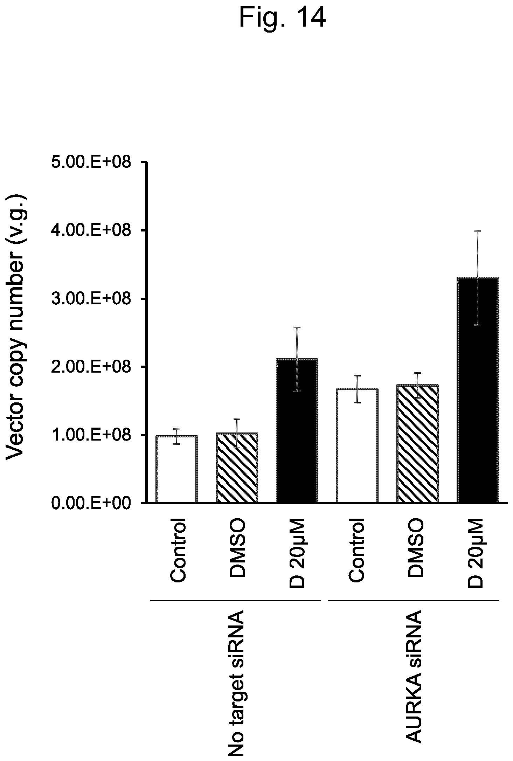

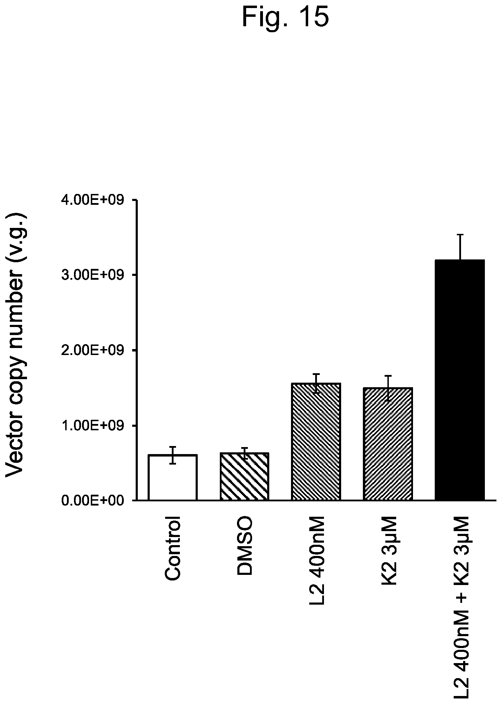

Figure 13 shows the results of Combination Example 1, Figure 14 shows the results of Combination Example 2, and Figure 15 shows the results of Combination Example 3. In Figures 13 to 15, the vertical axis represents the amount of production of AAV vectors (v.g.), and the horizontal axis represents types and concentration of chemical substances.

As shown in Figure 13, effects of enhancing production of AAV vectors were improved to a significant extent with the use of the inhibitor against the serine/threonine kinase Chk1 in combination with the inhibitor against the sarcoplasmic reticulum Ca²⁺ -ATPase, compared with the effects achieved with the use of either of the agents by itself. The results demonstrate that greater effects of enhancing production of AAV vectors can be expected with the use of the inhibitor against the serine/threonine kinase Chk1 in combination with the inhibitor against the sarcoplasmic reticulum Ca²⁺ -ATPase without inhibiting the effects of enhancing production of AAV vectors of the other agent.

As shown in Figure 14, effects of enhancing production of AAV vectors were improved to a significant extent with the use of the inhibitor against the Aurora kinase in combination with the inhibitor against the sarcoplasmic reticulum Ca²⁺ -ATPase, compared with the effects achieved with the use of either of the agents by itself. The results demonstrate that greater effects of enhancing production of AAV vectors can be expected with the use of the inhibitor against the Aurora kinase in combination with the inhibitor against the sarcoplasmic reticulum Ca²⁺ -ATPase without inhibiting the effects of enhancing production of AAV vectors of the other agent.

As shown in Figure 15, effects of enhancing production of AAV vectors were improved to a significant extent with the use of the inhibitor against the Aurora kinase in combination with the inhibitor against the serine/threonine kinase Chk1, compared with the effects achieved with the use of either of the agents by itself. The results demonstrate that greater effects of enhancing production of AAV vectors can be expected with the use of the inhibitor against the Aurora kinase in combination with the inhibitor against the serine/threonine kinase Chk1 without inhibiting the effects of enhancing production of AAV vectors of the other agent.

### [Example 3]

In the present example, effects of enhancing production of AAV vectors achieved with the use of chemical substances different from the chemical substances tested in Example 1 were measured in the same manner as in Example 1. Table 2 shows the chemical substances tested in the present example.

**[Table 2]**

| | Chemical substance used | Manufacturer | Concentration |
|---|---|---|---|
| Chemical substance D3 | Suramin sodium salt | Calbiochem | 100, 200, 400, 600, 800, 1000 µg/ml |
| Chemical substance L3 | Barasertib | Selleckchem.com | 1, 3, 10, 25, 50 µM |
| Chemical substance L4 | SP-96 | Selleckchem.com | 100, 250, 500, 1000, 2000 nM |
| Chemical substance L5 | MLN8054 | Selleckchem.com | 0.5, 1, 2, 4, 8 µM |

### [Results of experiment]

### 1) Chemical substance D3 (Suramin sodium salt)

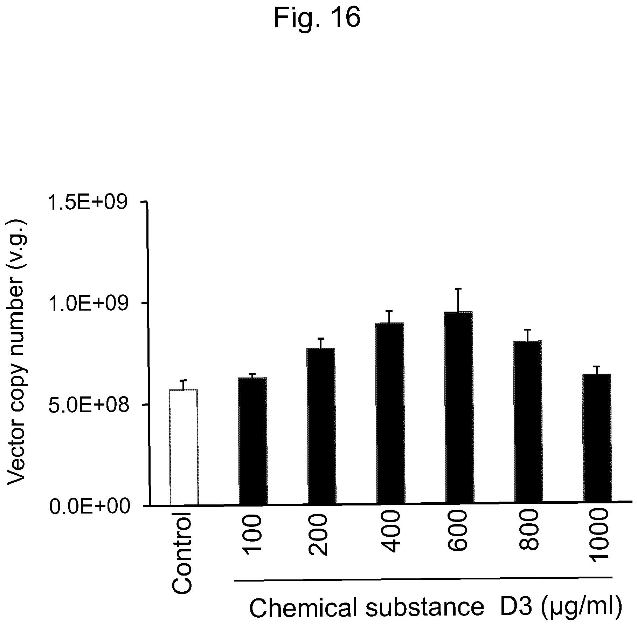

Figure 16 shows the results of measuring the effects of enhancing production of rAAV vectors achieved with the use of a chemical substance D3. In Figure 16, the vertical axis represents the amount of production of AAV vectors (v.g.), and the horizontal axis represents the concentration of the chemical substance. As shown in Figure 16, the chemical substance D3 is an analog of the chemical substance D tested in Example 1, and the chemical substance D3 has effects of enhancing the amount of production of AAV vectors as with the chemical substance D. In addition, the chemical substance D3 was found to exert greater effects of enhancing production of AAV vectors in a range of concentration from 200 to 800 µg/ml.

### 2) Chemical substance L3 (Barasertib)

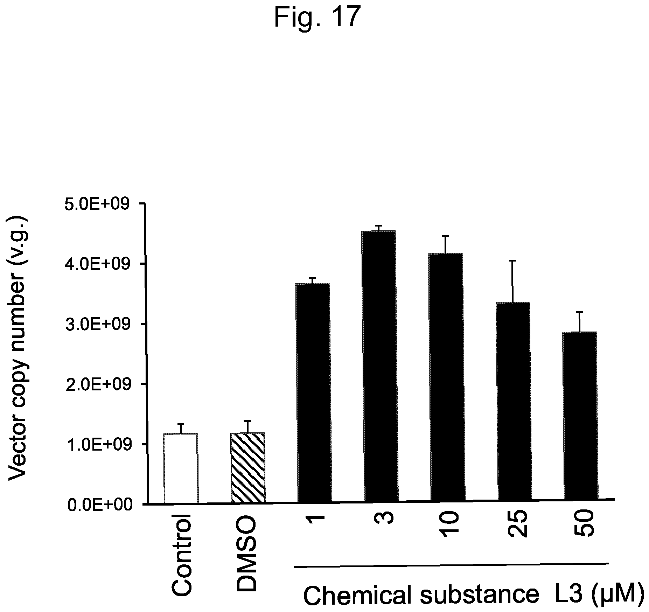

Figure 17 shows the results of measuring the effects of enhancing production of rAAV vectors achieved with the use of the chemical substance L3. In Figure 17, the vertical axis represents the amount of production of AAV vectors (v.g.), and the horizontal axis represents the concentration of the chemical substance. As shown in Figure 17, the chemical substance L3 having specific inhibitory activity against AURKB has effects of enhancing production of AAV vectors, and, in particular, greater effects of enhancing the amount of production of AAV vectors are observed in a range of concentration from 1 to 10 µM.

### 3) Chemical substance L4 (SP-96)

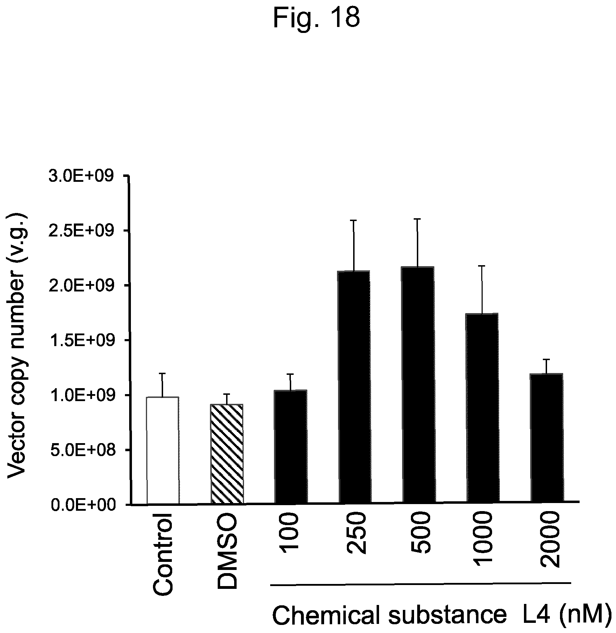

Figure 18 shows the results of measuring the effects of enhancing production of rAAV vectors achieved with the use of the chemical substance L4. In Figure 18, the vertical axis represents the amount of production of AAV vectors (v.g.), and the horizontal axis represents the concentration of the chemical substance. As shown in Figure 18, the chemical substance L4 having specific inhibitory activity against AURKB has effects of enhancing production of AAV vectors, and, in particular, greater effects of enhancing the amount of production of AAV vectors are observed in a range of concentration from 250 to 1000 nM.

### 4) Chemical substance L5 (MLN8054)

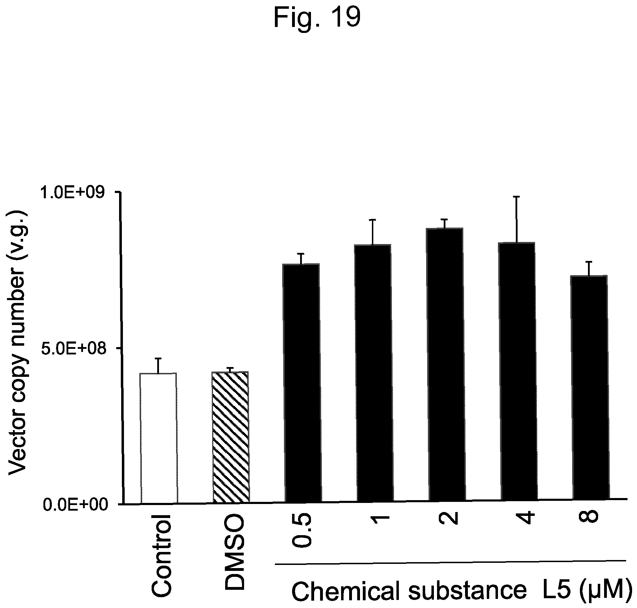

Figure 19 shows the results of measuring the effects of enhancing production of rAAV vectors achieved with the use of the chemical substance L5. In Figure 19, the vertical axis represents the amount of production of AAV vectors (v.g.), and the horizontal axis represents the concentration of the chemical substance. As shown in Figure 19, the chemical substance L5 having inhibitory activity against AURKA and AURKB has effects of enhancing production of AAV vectors, and, in particular, greater effects of enhancing the amount of production of AAV vectors are observed in a range of concentration from 1 to 4 µM.

### [Example 4]

In the present example, effects of enhancing production of AAV vectors were measured in the same manner as in Example 1 with the use of siRNA capable of knocking down the human AURKB gene, which was different from siRNA tested in Example 1. In the present example, siRNA targeting the target human AURKB gene was purchased (siGENOME Human AURKB siRNA-SMARTpool, Horizon Discovery Group Company).

In the present example, the expression level of the human AURKB gene and the amount of production of the rAAV vectors were measured in the same manner as in Example 1, except for the use of the primer sequences targeting human AURKB (forward primer: 5'-TGCTTTGCTATGAGCTGCTG-3' (SEQ ID NO: 7); reverse primer: 5'-GCCTGAGCAGTTTGGAGATG-3' (SEQ ID NO: 8)).

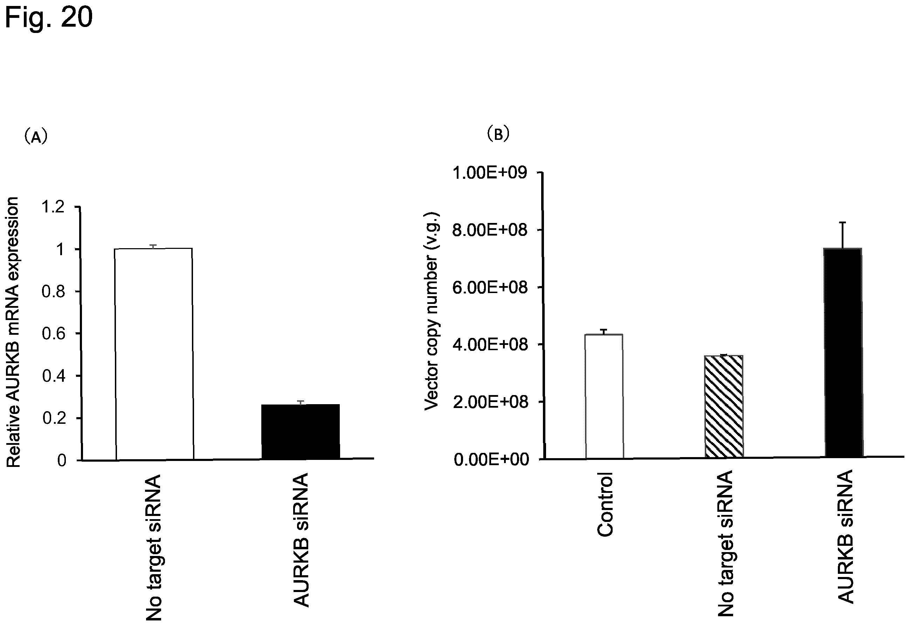

Figure 20 shows the results of evaluating the AAV vector-producing ability by knocking down the human AURKB gene by siRNA. Figure 20 (A) shows the expression level of the human AURKB gene treated with siRNA targeting the human AURKB gene or no-target siRNA and Figure 20 (B) shows the amount of production of rAAV vectors when treated with siRNA targeting the human AURKB gene or no-target siRNA. As shown in Figure 20, the effects of enhancing production of AAV vectors were observed by knocking down the human AURKB gene, which is a target molecule of the chemical substances L3 and L4, by siRNA. The results shown in Figure 20 demonstrate that the effects of enhancing the amount of production of rAAV vectors are achieved by inhibiting the human AURKB gene as with the case of the effects of enhancing production of rAAV vectors achieved by inhibiting the human AURKA gene with the use of the chemical substance L (Figure 11). The results also demonstrate that the effects of enhancing production of rAAV vectors can be achieved by inhibiting the human AURKB gene with the use of a substance other than the chemical substances L3 and L4.

### [Comparative Example]

WO 2021/138387 discloses a technique of cell cycle control in optimizing the production efficiency of gene transfer vectors. WO 2021/138387 discloses the addition of components, such as dehydroascorbic acid, hydroxyurea, aphidicolin, PD 0332991 HCI, Dinaciclib, AT7519, BS-181 HCI, AZD7762, PF 477736, LY2603618, CHIR-124, and MK-8776, to a medium for cell cycle control. In particular, WO 2021/138387 discloses in the examples that the expression levels of the genes encoded by viral vectors are increased when ascorbic acid is added to the medium.

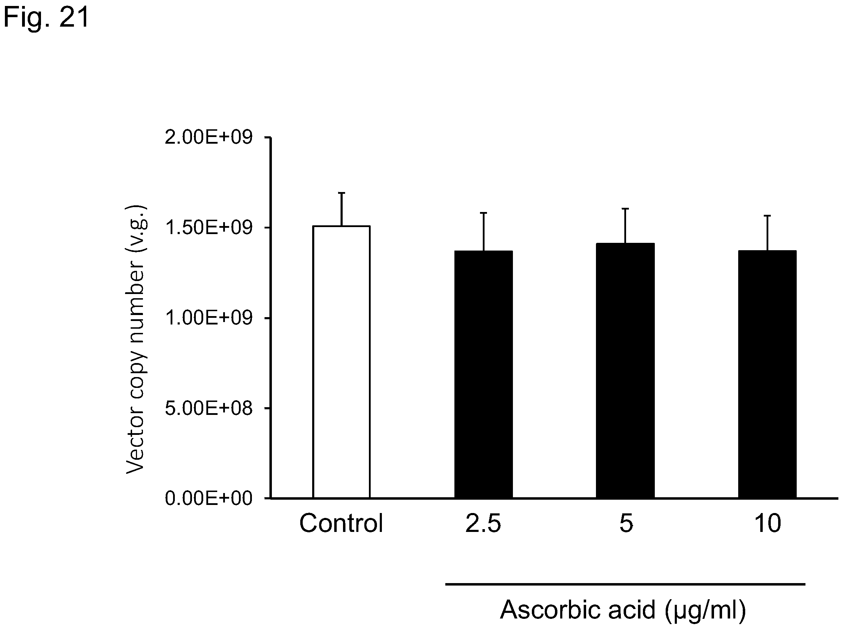

In the present comparative example, effects of enhancing production of AAV vectors were measured in the same manner as in Example 1, except for the use of ascorbic acid (2.5, 5, or 10 µg/ml) disclosed in the examples of WO 2021/138387. Figure 21 shows the results of measurement. In Figure 21, the vertical axis represents the amount of production of AAV vectors (v.g.), and the horizontal axis represents the concentration of ascorbic acid.

As shown in Figure 21, no significant differences were observed in the amount of production of rAAV vectors between the case involving the use of a medium supplemented with ascorbic acid and the control. The results of the comparative example demonstrate that, unlike the inhibitor against the sarcoplasmic reticulum Ca²⁺ -ATPase, the inhibitor against the serine/threonine kinase Chk1, and the inhibitor against the Aurora kinase demonstrated in Examples 1 to 4, ascorbic acid does not have effects of enhancing production of rAAV vectors.

## Claims

1. An agent for enhancing production of a viral vector comprising, as a major ingredient, an inhibitor against a protein involved in the DNA damage response.

2. The agent for enhancing production of a viral vector according to claim 1, which is used for production of a viral vector using viral a vector-producing cell.

3. The agent for enhancing production of a viral vector according to claim 1, which comprises, as the inhibitor against a protein involved in the DNA damage response, one or more substances selected from the group consisting of an inhibitor against the sarcoplasmic reticulum Ca²⁺-ATPase, an inhibitor against the serine/threonine kinase Chk1, and an inhibitor against the Aurora kinase.

4. The agent for enhancing production of a viral vector according to claim 3, wherein the inhibitor against the sarcoplasmic reticulum Ca²⁺ -ATPase is cyclopiazonic acid, Thapsigargin, or an analog thereof.

5. The agent for enhancing production of a viral vector according to claim 3, wherein the inhibitor against the serine/threonine kinase Chk1 is PF-477736, Rabusertib, AZD7762, or an analog thereof or an RNAi molecule targeting the Chk1 gene.

6. The agent for enhancing production of a viral vector according to claim 3, wherein the inhibitor against the Aurora kinase is CYC116, Alisertib, or an analog thereof or an RNAi molecule targeting the Aurora kinase gene.

7. A method for producing a viral vector comprising: a step of culturing a viral vector-producing cell in which a protein involved in the DNA damage response is inhibited; and a step of collecting the viral vector from the cultured cell.

8. The method for producing a viral vector according to claim 7, wherein, in the step of culturing the cell, the cell is cultured in a medium supplemented with the agent for enhancing production of the viral vector according to any one of claims 1 to 6.

9. The method for producing a viral vector according to claim 7, wherein the cell lacks at least one gene selected from the group consisting of the sarcoplasmic reticulum Ca²⁺ -ATPase gene, the serine/threonine kinase Chk1 gene, and the Aurora kinase gene.
